# EUROPEAN PATENT APPLICATION

(11) **EP 4 230 263 A1**
(43) Date of publication of application: **23.08.2023**
(21) Application number: 22735066.7
(22) Date of filing: 28.01.2022
(51) Int. Cl.: A61P 1/02, A61Q 11/00, A61K 8/67, A61K 8/49, A23G 4/12, A23G 4/06

(54) **ORAL CARE ADDITIVE, ORAL CARE COMPOSITION, PREPARATION METHOD, KIT, AND APPLICATION OF ORAL CARE ADDITIVE**

(30) Priority: 17.12.2021 CN 202111550053
(71) Applicant: Bioright Worldwide Company Ltd., Road Town, Tortola (VG)
(72) Inventor: POON, Wing Keung, Shatin, New Territories (HK); LO, Kam Chun, Shatin, New Territories (HK)
(74) Representative: Cohausz & Florack
(86) International application number: PCT/CN2022/074521
(87) International publication number: WO 2023/108866

(57) **Abstract**

Provided herein are an oral care additive, an oral care composition, a method of preparing the same, an oral care kit and use thereof. The main ingredient of the oral care additive contains one or more of nicotinic acid, nicotinamide or derivatives thereof to supplement and increase the level of β-nicotinamide adenine dinucleotide in the oral cavity, and various vitamin and coenzyme may be included as well to complement the function of the main ingredient. The additives and the application methods of the present invention retain the functionality of the existing oral care products while adding new efficacy, thereby providing a more comprehensive oral health management experience for the user.

## Description

### TECHNICAL FIELD

The present invention belongs to the personal care field and specifically relates to an oral care additive, an oral care composition, a preparation method, a kit and uses thereof, and in particular, to the use of additives comprising β-nicotinamide mononucleotide as the main ingredient in current and new oral care products to increase their efficacy.

### BACKGROUND

Oral diseases, which mainly include malocclusion, dental caries, inflamed and bleeding gums, gum recession and periodontitis, have been listed by China and the World Health Organization as one of the top five priority chronic diseases, alongside traditional chronic diseases such as cardio-cerebral vascular diseases, cancers and diabetes. The number of patients with oral diseases in China grows year by year, and according to the data in 2018 the number of patients already exceeded 694 million, reflecting a rather serious situation. Among the various oral diseases, the dental caries, gum inflammation and bleeding are the most common symptoms, mostly due to poor oral cleaning, resulting in the long-term accumulation of plaque on the teeth and gums in which the bacteria will produce toxins to stimulate the tissue and cause disease. Oral hygiene is an important part of the prevention and treatment of oral diseases, and China has been actively promoting and educating the importance of oral hygiene for many years. In addition to instilling the importance of daily tooth brushing in young children, a lot of resources are invested to promote health care knowledge and improve behaviors among the population, but the problem of increasing oral diseases, especially among Chinese adults, has not been addressed.

The aging population and smoking are also major causes of the oral diseases, especially as China's aging population becomes more and more serious. When the elderly population over 60 years old accounts for a quarter of the overall population in 2030, the number of patients with oral diseases will remain high and the burden on the country's oral health care will become even heavier. The oral diseases will make it inconvenient for patients to chew. Also, recent studies have found an association between the periodontal disease and other chronic diseases including diabetes, cancers and cardiovascular disease. Therefore, oral health can be recognized as the frontline of public health development. If the severity and relevance of the dental diseases are not addressed, the health of the general public will inevitably regress and, given the prevalence of oral disease, it will certainly become a gap in China's health care in the foreseeable future, posing a significant risk to the interests of the population and society unless it is fixed now.

The main countermeasures for oral care nowadays are to focus on oral cleaning, including adding various substances into oral care products to eliminate microorganisms in the oral cavity and inhibit their reproduction rate to reduce the formation of plaque and tartar, such as brushing teeth properly with fluoride toothpaste every morning and night, using mouthwash, flossing after eating to remove the residue in mouth, and regular dental checkups at dental clinics. These countermeasures have positive effects on prevention of oral diseases and should be promoted and implemented continuously. However, these countermeasures solely focus on prevention and treatment, and they can only slow the conditions of adults, especially those who are already suffering from oral diseases, without producing substantial improvement. And, for the patients with oral diseases that are not caused by oral bacteria, such as those with poorly aligned teeth, teeth grinding, or gum or capillary recession caused by long-term smoking, they are not very effective. In addition, recent studies have found that the oral diseases, particularly the inflammation and bleeding of gum, the periodontal disease and toothbone degeneration due to gum and capillary atrophy, may be caused by an increase in inflammatory cytokines in the gum tissue. When the gum inflammation and periodontal disease occur, the immune system of body produces reactive oxidants and inflammatory cytokines to combat the invasion of bacteria and pathogens, but these substances also damage the gingival tissue. Studies have found that activating the longevity protein gene (Sirtuin 1) of the gingival tissue and suppressing the continued production of inflammatory cytokines is an effective response to these conditions.

For many years, oral care products have only focused on the development of the function in bacteria reduction or the addition of chemical substances, and physical measures in response to the problems such as tooth and gingival sensitivity have been old-fashioned, such that the functional development of oral care products has remained stagnant for many years. Even though the general public has become increasingly aware of oral care, the number of the patients having oral diseases has increased year after year, posing an unprecedented public health challenge in China.

Therefore, there is still a need for new (preferably improved in at least one respect) oral care additives and oral care compositions using such additives.

### SUMMARY

In order to solve the above problems, the present invention provides an oral care additive, use thereof in oral care compositions, an oral care composition, a method of preparing the oral care composition, a method of use, and in particular the use of the additive by adding to an existing oral care product to enhance the efficacy of the product (e.g., to treat, prevent or relieve an oral disease such as a periodontal disease or inflammation of the gums, etc.). The present invention also proposes applications and methods for products comprising the additive as the main ingredient.

The proposed oral care additive is based on β-nicotinamide mononucleotide as the main ingredient, which can be added to the existing oral care products in a combined manner to enhance the level of β-nicotinamide adenine dinucleotide in the gum tissue and at the same time exert the original efficacy of the product. The present invention also proposes novel oral care products with the additive as the main ingredient, including a chewing gum and an oral gelling agent containing β-nicotinamide mononucleotide, to provide a suitable platform for the use of the additive combination and to provide convenience of use for users with different oral health conditions.

Specifically, the present invention provides:
1. An oral care additive comprising at least one main ingredient selected from nicotinic acid or a derivative thereof and nicotinamide or a derivative thereof.
2. The oral care additive according to any of the above, wherein the nicotinic acid or derivative thereof and the nicotinamide or derivative thereof are selected from β-nicotinic acid riboside and a salt thereof, β-nicotinic acid mononucleotide and a salt thereof, oxidized β-nicotinic acid adenine dinucleotide and a salt thereof, reduced β-nicotinic acid adenine dinucleotide and a salt thereof, oxidized β-nicotinic acid adenine dinucleotide and a salt thereof, reduced β-nicotinic acid adenine dinucleotide phosphate and a salt thereof, oxidized β-nicotinic acid adenine dinucleotide phosphate and a salt thereof, β-nicotinamide riboside and a salt thereof, β-nicotinamide mononucleotide and a salt thereof, oxidized β-nicotinamide adenine dinucleotide and a salt thereof, reduced β-nicotinamide adenine dinucleotide and a salt thereof, oxidized β-nicotinamide adenine dinucleotide phosphate and a salt thereof or reduced β-nicotinamide adenine dinucleotide phosphate and a salt thereof.
3. The oral care additive according to any of the above, wherein the oral care additive further comprises an auxiliary ingredient selected from at least one of vitamin and coenzyme.
4. The oral care additive according to any of the above, wherein the vitamin is selected from at least one of vitamin A, vitamin B, vitamin C, vitamin D and vitamin K.
5. The oral care additive according to any of the above, wherein the coenzyme is selected from at least one of glutathione or derivatives thereof, adenosine triphosphate and derivatives thereof, pyrroloquinoline quinone or derivatives thereof, adenosylmethionine or derivatives thereof, coenzyme A or derivatives thereof, coenzyme Q class or derivatives thereof.
6. The oral care additive according to any of the above, wherein the derivative is β-nicotinic acid riboside or a salt thereof, β-nicotinic acid mononucleotide or a salt thereof, β-nicotinamide riboside or a salt thereof, β-nicotinamide mononucleotide or a salt thereof, oxidized β-nicotinamide adenine dinucleotide or a salt thereof, or reduced β-nicotinamide adenine dinucleotide or a salt thereof;
   wherein the vitamin is vitamin B, vitamin C or vitamin K; and
   wherein the coenzyme is glutathione or a derivative thereof, or pyrroloquinoline quinone or a derivative thereof.
7. The oral care additive according to any of the above, wherein the oral care additive is in a solid, liquid or gaseous form.
8. The oral care additive according to any of the above, wherein the solid is selected from at least one of powder, paste, gel and crystals, optionally, the liquid is selected from at least one of solution, slurry, essential oil and non-Newtonian fluid; and optionally, the said gaseous form is selected from liquefied spray.
9. The oral care additive according to any of the above, wherein the solid is selected from gel, the liquid is selected from solution, and the gaseous form is selected from liquefied spray.
10. The oral care additive according to any of the above, wherein the main ingredient is present in an amount of 0.1-100% (w/w), preferably 0.1-90% (w/w), and more preferably 0.1-50% (w/w) based on a total weight of the oral care additive.
11. The oral care additive according to any of the above, wherein the weight ratio of the main ingredient : the vitamin : the coenzyme is (0.1-99%) : (0.1-99%) : (0.1-99%), preferably (0.1-75%) : (0.1-12.5%) : (0.1-12.5%), and more preferably (0.1-50%) : (0.1 -25%) : (0.1-25%).
12. An oral care composition comprising the oral care additive according to any of Items 1-11.
13. The oral care composition according to any of the above, wherein the oral care additive is present in an amount of 0.1-50% (w/w), preferably 0.1-25% (w/w), and more preferably 0.3-15% (w/w) based on a total weight of said oral care composition.
14. The oral care composition according to any of the above, wherein the oral care composition is at least one selected from toothpaste, mouthwash, oral spray and tooth cleaning powder.
15. The oral care composition according to any of the above, wherein the oral care composition is a chewing gum or oral gelling agent.
16. The oral care composition according to any of the above, wherein the oral care composition is in a solid, liquid or gaseous form.
17. The oral care composition according to any of the above, wherein the chewing gum further comprises at least one of a resin, a wax, a phospholipid, a flavoring agent and an elastomer; and
   preferably, the oral gelling agent further comprises glycerin, sorbitol, tragacanth gum, sodium benzoate and peppermint oil.
18. A method of preparing an oral care composition, comprising the steps of
   providing an oral care additive comprising at least one main ingredient selected from nicotinic acid or a derivative thereof and nicotinamide or a derivative thereof; and
   adding the oral care additive to a carrier that facilitates absorption of the oral care additive into an oral gingival tissue.
19. The method according to any of the above, wherein said carrier is at least one of a toothpaste, a mouthwash, an oral spray, a tooth cleaning powder, a chewing gum and an oral gelling agent.
20. The method according to any of the above, wherein the oral care additive further comprises an auxiliary ingredient selected from at least one of vitamin and coenzyme.
21. The method according to any of the above, wherein the nicotinic acid or derivative thereof and the nicotinamide or derivative thereof are selected from β-nicotinic acid riboside and a salt thereof, β-nicotinic acid mononucleotide and a salt thereof, oxidized β-nicotinic acid adenine dinucleotide and a salt thereof, reduced β-nicotinic acid adenine dinucleotide and a salt thereof, oxidized β-nicotinic acid adenine dinucleotide and a salt thereof, reduced β-nicotinic acid adenine dinucleotide phosphate and a salt thereof, oxidized β-nicotinic acid adenine dinucleotide phosphate and a salt thereof, β-nicotinamide riboside and a salt thereof, β-nicotinamide mononucleotide and a salt thereof, oxidized β-nicotinamide adenine dinucleotide and a salt thereof, reduced β-nicotinamide adenine dinucleotide and a salt thereof, oxidized β-nicotinamide adenine dinucleotide phosphate and a salt thereof or reduced β-nicotinamide adenine dinucleotide phosphate and a salt thereof.
22. An oral care kit comprising the oral care composition according to any of the above and a dental gel brace.
23. Use of the oral care additive according to any of the above in the preparation of an oral care composition for treatment, prevention or relief of an oral disease.

The present invention has the following features and positive effects.
1. The present invention proposes an oral care additive, use thereof and the related methods, in which one or more of nicotinic acid or derivatives thereof or nicotinamide or derivatives thereof (preferably β-nicotinamide mononucleotide and oxidized β-nicotinamide adenine dinucleotide phosphate and salts thereof) is/are the main ingredient(s) for replenishing and enhancing the level of β-nicotinamide adenine dinucleotide in the gingival tissue. The use of the composition in the oral health care is a new breakthrough in that the oral care products are no longer limited to the single function of maintaining oral cleanliness or relieving tooth sensitivity symptoms.
2. All substances of the oral care additive proposed by the present invention are natural and harmless and present in all living organisms, safe and reliable, and would not cause adverse reactions and effects on the oral health of general users, and are suitable for daily use by people of any age.
3. The invention proposes to add the oral care additive to the existing oral care products such as toothpaste and mouthwash, which is easy to perform. The additive combination will not react scientifically with the ingredients in the oral care products, so the additive combination will not affect or reduce the original function and efficacy of the oral care products. When using the oral care product, the user can clean mouth and at the same time absorb the ingredients of the additive combination in the process to replenish and enhance the level of β-nicotinamide adenine dinucleotide in the gum, providing a more comprehensive oral care in a two-pronged manner without the need of additional time.
4. One of the novel oral care compositions proposed by the present invention is a chewing gum containing the oral care additive. This product is suitable for the user with generally normal chewing ability to replenish and enhance the level of β-nicotinamide adenine dinucleotide in the gingiva by chewing the gum anytime and anywhere. When the user chews the gum, the additives in the gum are released, and since the chewing process generally lasts a relatively long period, it increases the chance of direct absorption by the capillaries in the gingiva. This practice not only makes effective use of the physical properties of chewing gum to upload a large amount of additives, but also takes advantage of the habit of chewing gum. That is, the gum adheres to the gingiva when being chewed constantly in the mouth, and the additives in the gum can be absorbed by the capillaries in the gingival tissue in the most direct and stable way, which is more direct and long-lasting than other methods of use.
5. Another new oral care composition proposed by the present invention is an oral gel containing the oral care additive. This product is suitable for people who have poor chewing ability and use dentures. These people are mostly the aged persons or patients having severe periodontal disease, who need more urgently to supplement and enhance the level of nicotinamide adenine dinucleotide to promote gum health, reduce gum inflammation and promote the growth of gum capillaries to avoid gum recession. Such functions are lacked in the existing oral care products, and the present product is especially designed for this type of people. The oral gel is primarily water-based, and can be added with natural calming ingredients such as peppermint to give the user a soothing effect. The user simply injects the oral gel into the braces and puts them into the mouth cavity, such that the oral gel directly contacts the gum surface to create the most direct conditions for the gums to absorb directly. Also, the water-based gel allows the user to apply it at a chilled to holding temperature, allowing the user to make the most effective choice for their own experience.

### BRIEF DESCRIPTION OF DRAWINGS

FIG. 1 illustrates the results of stability studies of Example 6 (shown in the figure as oral care additive) and Example 7 (shown in the figure as β-nicotinamide mononucleotide) over time.
FIG. 2 illustrates the growth of oral tissue cells in a medium without an oral care additive and added with the oral care additive of Example 2 at different doses.

### DETAILED DESCRIPTION OF EMBODIMENTS

The following is a further explanation of the invention through the description of specific embodiments, but it is not a limitation thereto. A person skilled in the art can make various modifications or improvements according to the basic concept herein, which fall in the scope of the present disclosure without departing from the basic concept herein.

Oral diseases have become the most important public health problem in China, and the number of people suffering from dental caries and periodontal disease has exceeded one-third of the overall population, which has a tendency to increase gradually. Together with the existing problem of aging population, it appears that in the foreseeable years the oral diseases will rise to the top of the chronic diseases in China, and will make a heavy burden over the China's health care. Therefore, the society should pay attention to the importance of the oral diseases. The importance of oral health has been raised in the "Healthy China 2030 Plan" and the "Medium and Long-term Plan for the Prevention and Control of Chronic Diseases in China (2017-2025)", which have been implemented to further strengthen the oral health promotion and enhance public action and motivation for oral health. The above policies and measures have been gradually put in place, especially in the oral health of adolescents, but the number of persons with oral diseases in China is still high, and it is desired that a new thought be injected into this accumulating public health problem.

With the rapid development of science and technology in recent years, the scientific community has been conducting more extensive research on oral health and diseases, especially on the importance of β-nicotinamide adenine dinucleotide to oral health.

β-nicotinamide adenine dinucleotide is an important coenzyme in living organisms, which is not only responsible for regulating various biological functions in the body, but also for activating longevity protein genes and suppressing inflammatory cytokines, so that it is very important to use substances that can enhance the level of β-nicotinamide adenine dinucleotide in the gingival tissue for oral care. β-nicotinamide mononucleotide is a direct precursor to β-nicotinamide adenine dinucleotide and enzymatically converted to β-nicotinamide adenine dinucleotide in the cells. β -nicotinamide mononucleotide is highly stable and safe, and is readily absorbed by the body, making it an ideal substance for raising the level of β -nicotinamide adenine dinucleotide in the gum.

In addition, direct administration of other nicotinic acid or derivatives thereof and nicotinamide or derivatives thereof has the similar function, which may include at least one of β-nicotinic acid riboside and a salt thereof, β-nicotinic acid mononucleotide and a salt thereof, oxidized β-nicotinic acid adenine dinucleotide and a salt thereof, reduced β-nicotinic acid adenine dinucleotide and a salt thereof, oxidized β-nicotinic acid adenine dinucleotide and a salt thereof, reduced β-nicotinic acid adenine dinucleotide phosphate and a salt thereof, oxidized β-nicotinic acid adenine dinucleotide phosphate and a salt thereof, β-nicotinamide riboside and a salt thereof, β-nicotinamide mononucleotide and a salt thereof, oxidized β-nicotinamide adenine dinucleotide and a salt thereof, reduced β-nicotinamide adenine dinucleotide and a salt thereof, oxidized β-nicotinamide adenine dinucleotide phosphate and a salt thereof or reduced β-nicotinamide adenine dinucleotide phosphate and a salt thereof.

The present inventors noted that patients with oral diseases such as gingivitis and periodontal diseases are concentrated in the adult to the elder group, and that these patients tend to have increasingly heavier conditions over age, even affecting their chewing ability and reducing their quality of life. The present inventors have recognized that this aging situation is closely related to the recent scientific studies confirming that the level of β-nicotinamide adenine dinucleotide in the human body gradually decreases with age, and have found that recent studies also indicate that β-nicotinamide adenine dinucleotide plays a protective role in gingival inflammation and activates the longevity protein gene 1 in the gums to inhibit the damage to the gingival tissues by the active oxidants produced in the periodontal disease. Therefore, the present inventors believe that the level of β-nicotinamide adenine dinucleotide in the gums is essential for the oral health. The inventors believe that the level of β-nicotinamide adenine dinucleotide in these people is relatively low and should be raised as soon as possible, and that it is necessary to have β-nicotinamide adenine dinucleotide absorbed directly in the gums. According to recent scientific studies, the absorption of nicotinamide or derivatives thereof can raise the level of β-nicotinamide adenine dinucleotide in living beings, and it is indicated that the use of β-nicotinamide mononucleotide, the most precursor of β-nicotinamide adenine dinucleotide, is desirable in terms of both efficacy and safety.

Therefore, the present inventors investigated the possibility of applying such substances. The results showed that β-nicotinamide mononucleotide is easily absorbed by the body and can be rapidly converted into β-nicotinamide adenine dinucleotide in vivo to elevate the level thereof in the body. The studies also showed that increasing the level of β-nicotinamide adenine dinucleotide has a significant effect on the metabolism, physiological clock and physical functions of the body. Among others, the studies have demonstrated that boosting β-nicotinamide adenine dinucleotide by β-nicotinamide mononucleotide can promote capillary growth in muscles to improve function, which can be used to promote capillary growth in the gums to prevent tooth loss caused by the gum recession in periodontal disease. β-nicotinamide mononucleotide is a natural substance with high stability and has been proven to be safe for human body, which has been taken by people in various countries with good feedback. Therefore, the inventors believed that β-nicotinamide mononucleotide is the most suitable substance as the main ingredient in the additive. β-nicotinamide adenine dinucleotide is an important coenzyme in the body, and all cells and tissues in the body depend on its presence to carry out various types of physiological reactions, but it is not substantially effective to deal with oral problems by relying on oral administration of β -nicotinamide mononucleotide. Thus, β-nicotinamide mononucleotide is required to enter the gums and be absorbed by the capillaries therein in a more direct way, in order to directly raise the level of β-nicotinamide adenine dinucleotide in the gums. At the same time, the inventors believe that the applications and methods of enhancing the level of β-nicotinamide adenine dinucleotide in the gums should be user-friendly and complementary to the existing oral cleaning habits.

Therefore, the present invention proposes an oral care additive, an oral care composition, uses and methods thereof to provide a new means based on traditional oral care, increase the efficacy of the existing oral care products and provide new applications and methods so that the new scientific results can be practically applied to the general public to integrate oral cleaning and an increase in the level of β-nicotinamide adenine dinucleotide in the gums, creating more favorable oral health conditions in line with the general public's need for personal health and public health benefits.

The oral care additive and use thereof herein break the currently inherent thoughts and patterns of oral health care that is biased toward oral cleaning, and incorporate the existing oral care habits with the new thought of enhancing β-nicotinamide adenine dinucleotide levels to bring a more comprehensive oral health experience to the public and create a more solid foundation for public health in China.

In one aspect, the present invention provides an oral care additive comprising at least one main ingredient selected from nicotinic acid or a derivative thereof and nicotinamide or a derivative thereof.

The nicotinic acid or the derivative thereof and the nicotinamide or the derivative thereof may be selected from at least one of β-nicotinic acid riboside and a salt thereof, β-nicotinic acid mononucleotide and a salt thereof, oxidized β-nicotinic acid adenine dinucleotide and a salt thereof, reduced β-nicotinic acid adenine dinucleotide and a salt thereof, oxidized β-nicotinic acid adenine dinucleotide and a salt thereof, reduced β-nicotinic acid adenine dinucleotide phosphate and a salt thereof, oxidized β-nicotinic acid adenine dinucleotide phosphate and a salt thereof, β-nicotinamide riboside and a salt thereof, β-nicotinamide mononucleotide and a salt thereof, oxidized β-nicotinamide adenine dinucleotide and a salt thereof, reduced β-nicotinamide adenine dinucleotide and a salt thereof, oxidized β-nicotinamide adenine dinucleotide phosphate and a salt thereof, or reduced β-nicotinamide adenine dinucleotide phosphate and a salt thereof.

Preferably, the main function of the oral care additive is the elevation and replenishment of the level of β-nicotinamide adenine dinucleotide in the oral cavity, and the oral care additive combination contains at least one functional substance, either direct or indirect, which may be one or more nicotinic acid or the derivative thereof and nicotinamide or the derivative thereof. The one or more nicotinic acid or the derivative thereof may comprise nicotinic acid, β -nicotinic acid riboside and a salt thereof, β-nicotinic acid mononucleotide and a salt thereof, oxidized β-nicotinic acid adenine dinucleotide and a salt thereof, reduced β-nicotinic acid adenine dinucleotide and a salt thereof, oxidized β-nicotinic acid adenine dinucleotide and a salt thereof, reduced β -nicotinic acid adenine dinucleotide phosphate and a salt thereof, and oxidized β-nicotinic acid adenine dinucleotide phosphate and a salt thereof. The one or more nicotinamide or derivative thereof may comprise nicotinamide, β-nicotinamide riboside or a salt thereof, β-nicotinamide mononucleotide or a salt thereof, oxidized β-nicotinamide adenine dinucleotide and a salt thereof, reduced β-nicotinamide adenine dinucleotide and a salt thereof, oxidized β-nicotinamide adenine dinucleotide phosphate and a salt thereof, or reduced β-nicotinamide adenine dinucleotide phosphate and a salt thereof.

The sum of the weight percentages of the at least one of the direct or indirect functional substances shall be 0.1-100% (w/w), preferably 0.1-90% (w/w), and more preferably 0.1-50% (w/w) based on the whole oral care additive.

The direct or indirect functional substances in the oral care additive may preferably be β-nicotinamide mononucleotide, β-nicotinamide riboside, oxidized β-nicotinamide adenine dinucleotide, reduced β-nicotinamide adenine dinucleotide, β-nicotinic acid mononucleotide and β-nicotinic acid riboside, more preferably β-nicotinamide mononucleotide, β-nicotinamide riboside and oxidized β-nicotinamide adenine dinucleotide, and even more preferably β-nicotinamide mononucleotide.

Preferably, the oral care additive also comprises an auxiliary ingredient selected from at least one of vitamin and coenzyme.

The vitamin may be selected from at least one of vitamin A, vitamin B, vitamin C, vitamin D, and vitamin K.

The coenzyme may be selected from at least one of glutathione or a derivative thereof, adenosine triphosphate and a derivative thereof, pyrroloquinoline quinone or a derivative thereof, adenosylmethionine or a derivative thereof.

In a preferred embodiment of the oral care additive, the nicotinic acid or the derivative thereof and the nicotinamide or the derivative thereof are β-nicotinic acid riboside or a salt thereof, β-nicotinic acid mononucleotide or a salt thereof, β-nicotinamide riboside or a salt thereof, β-nicotinamide mononucleotide or a salt thereof, oxidized β-nicotinamide adenine dinucleotide or a salt thereof, or reduced β-nicotinamide adenine dinucleotide or a salt thereof; the vitamin is vitamin B, vitamin C, or vitamin K; and the coenzyme is at least one of glutathione or a derivative thereof, or pyrroloquinoline quinone or a derivative thereof, coenzyme A or a derivative thereof, coenzyme Q or a derivative thereof.

The oral care additive may be in solid, liquid or gaseous form. The solid form can be selected from a powder, a paste, a gel and a crystal. The liquid form can be selected from a solution, a slurry, an essential oil and a non-Newtonian fluid. The gaseous form can be selected from a liquefied spray.

According to an embodiment of the oral care additive herein, the at least one main ingredient may be present in an amount of 0.1-100% (w/w), preferably 0.1-90% (w/w), and more preferably 0.1-50% (w/w) based on the total weight of the oral care additive.

Preferably, the weight ratio of the main ingredient : the vitamin : the coenzyme is (0.1-99%) : (0.1-99%) : (0.1-99%), preferably (0.1-75%) : (0.1-12.5%) : (0.1-12.5%), and more preferably (0.1-50%) : (0.1-25%) : (0.1-25%).

The oral care additives herein have the advantage of high stability, neither chemically interacting with compounds in the existing oral care products nor affecting or reducing the established effectiveness of the products.

A second aspect herein provides an oral care composition comprising the oral care additive described above. In other words, the oral care composition herein may comprise at least one selected from nicotinic acid or a derivative thereof and nicotinamide or a derivative thereof.

In one embodiment, the oral care additive may be present in an amount of 0.1-50% (w/w), preferably 0.1-25% (w/w), and more preferably 0.3-15% (w/w) based on the total weight of the oral care composition.

In one embodiment, the oral care composition may be at least one selected from toothpaste, mouthwash, oral spray, and tooth cleaning powder. These are all the existing oral care products.

The oral care composition can also be a new oral care product, such as a chewing gum or an oral gelling agent.

The oral care additive may be in solid, liquid or gaseous form. The solid form can be selected from a powder, a paste, a gel and a crystal. The liquid form can be selected from a solution, a slurry, an essential oil and a non-Newtonian fluid. The gaseous form can be selected from a liquefied spray.

As a novel oral care product, the chewing gum may also comprise at least one of a resin, a wax, a phospholipid, a flavoring agent, and an elastomer. In one embodiment, the novel oral care product, i.e., the chewing gum containing the oral care additive provides a carrier for the oral care additive, which has the advantage of high loading and portability, and which can be securely adhered to the gingival surface of the user, forming favorable conditions that allow the gums and capillaries thereof to directly absorb the oral care additive combination released from the chewing gum. The substances are also released upon chewing by the user and absorbed by the gums and capillaries thereof using saliva as a mediator.

As a novel oral care product, the oral gelling agent may also comprise glycerin, sorbitol, tragacanth gum, sodium benzoate and peppermint oil. In one embodiment, the novel oral care product, i.e., the oral gelling agent containing the oral care additive provides a carrier for the oral care additive and is used in combination with braces. The carrier combined with the braces can cling to the gum surface and, allow the gums to absorb the substances in the oral care additive composition, which is suitable for those having poor chewing ability or wearing dentures and the patients with tooth loss.

The chewing gum and the oral gelling agent (or oral gel) containing the oral care additive can be flavored and colored differently to meet the needs of the users; wherein the oral gel can be used at 10-40 degrees Celsius.

In one embodiment, the present invention provides a chewing gum containing a combination of the oral care additive for the convenience of the general users. The chewing gum primarily contains resin, wax and elastomer as the main ingredients. The materials applied in the present invention are based on plant ingredients, the main components of which include ginseng tree sap, gum arabic and lecithin. The chewing gum has the same properties and functions as the ordinary chewing gum and can be decomposed naturally. Edible carrier used in combination with the oral care additive has several advantages: the ingredients of the chewing gum are all inert materials that do not react chemically with the substances in the oral care additive combination during production; 0.1-20% (w/w) of the oral care additive combination can be added to the chewing gum in solid or liquid form, and the ingredients of the oral care additive combination will not change and remain stable such that the content of the functional ingredients is maintained at 95% or more under normal storage conditions for a period of three months; and the chewing gum can be flavored with a variety of natural flavoring agents to create a suitable texture and taste for the user.

In another embodiment, the invention further creates favorable conditions for the absorption of β-nicotinamide mononucleotide (the main component of the additive) into the gums and the capillaries thereof by the user's habit of chewing gum. 1. The oral care additive composition contained in the chewing gum will be released into the user's gingivae each time he chews the gum. 2. Chewing the gum also stimulates saliva secretion, and the substances in the gum are absorbed into the body through saliva. 3. When chewing the gum, the gingivae will have frequent contact with the chewing gum and the user will naturally attach the chewing gum to the gingivae and teeth, thereby creating favorable conditions for prolonged contact with the gingival tissue and allowing the oral care composition to be absorbed directly by the gums and transported to the capillaries. The chewing gum containing the oral care additive composition is both portable and easy to use by the user, who can use it anytime and anywhere to replenish the level of β-nicotinamide adenine dinucleotide in the gums.

In another embodiment, the inventors have prepared another novel oral care product containing the oral care additive combination. The product is an oral gel for use by the persons with low chewing ability, the persons wearing dentures and the persons with oral diseases who have lost some of their teeth. 10% (w/w) of the oral care additive combination (6:2:2 of β-nicotinamide mononucleotide : S-acetylglutathione : vitamin C), 15% (w/w) of glycerol, 15% (w/w) of sorbitol, 2% (w/w) of tragacanth gum, 2.5% (w/w) of sodium benzoate, and 0.1% (w/w) of peppermint oil, based on a total weight of the finished product, are added to a container in that order. Pure water is added to a given volume and the mixture is stirred at 250 rpm for 12 hours to form a liquid paste state. The user injects the oral gel evenly into two dental gel braces before use, then puts the braces onto the upper and lower jaws, respectively, and closes his lips to prevent the braces from falling out. Ten minutes later, the user takes off the braces and rinses his mouth with water. The ingredients in the oral care additive combination of the oral gel are absorbed by the gums and capillaries thereof and the target application is achieved. The oral gel components are all inert materials and will not react chemically with the functional components of the oral care additive combination, but rather can stabilize the oral care additive combination at the same time. The stability thereof is as follows: the content of the oral care additive combination in the oral gel is maintained at 95% or more under normal storage conditions. The oral gel can be used after cooling down to not less than 10 degrees Celsius or holding to not more than 37 degrees Celsius, while its physical properties, functionality and stability will not be affected by changes in temperature.

The oral care additive according to the present invention has the advantage of high stability, neither chemically interacting with the compounds in the existing oral care products, nor affecting or reducing the established effectiveness of the products.

A third aspect herein provides a method for the preparation of an oral care composition, comprising the steps of:
providing an oral care additive comprising at least one main ingredient selected from nicotinic acid or a derivative thereof and nicotinamide or a derivative thereof; and
adding the oral care additive to a carrier that facilitates absorption of the oral care additive into a gingival tissue.

In one embodiment, the carrier is at least one of toothpaste, a mouthwash, an oral spray, a tooth cleaning powder, a chewing gum, and an oral gelling agent.

As described above, the oral care additive may also comprise an auxiliary agent selected from at least one of vitamin and coenzyme. The nicotinic acid or derivative thereof and the nicotinamide or derivative thereof may be selected from at least one of β-nicotinic acid riboside and a salt thereof, β-nicotinic acid mononucleotide and a salt thereof, oxidized β-nicotinic acid adenine dinucleotide and a salt thereof, reduced β-nicotinic acid adenine dinucleotide and a salt thereof, oxidized β-nicotinic acid adenine dinucleotide and a salt thereof, reduced β-nicotinic acid adenine dinucleotide phosphate and a salt thereof, oxidized β-nicotinic acid adenine dinucleotide phosphate and a salt thereof, β-nicotinamide riboside and a salt thereof, β-nicotinamide mononucleotide and a salt thereof, oxidized β-nicotinamide adenine dinucleotide and a salt thereof, reduced β-nicotinamide adenine dinucleotide and a salt thereof, oxidized β-nicotinamide adenine dinucleotide phosphate and a salt thereof, or reduced β-nicotinamide adenine dinucleotide phosphate and a salt thereof.

A fourth aspect herein provides an oral care kit comprising an oral care composition and a dental gel brace. The oral care composition may comprise at least one selected from nicotinic acid or a derivative thereof and nicotinamide or a derivative thereof.

According to one embodiment, an oral care additive combination is provided, wherein the oral care additive combination may be a combination of one or more nicotinic acid and a derivative thereof, nicotinamide and a derivative thereof, vitamin and coenzyme to serve as the main ingredients. The nicotinic acid or a derivative thereof includes nicotinic acid, β-nicotinic acid riboside and a salt thereof, β-nicotinic acid mononucleotide and a salt thereof, oxidized β-nicotinic acid adenine dinucleotide and a salt thereof, reduced β-nicotinic acid adenine dinucleotide and a salt thereof, oxidized β-nicotinic acid adenine dinucleotide phosphate and a salt thereof, and reduced β-nicotinic acid adenine dinucleotide phosphate and a salt thereof. The nicotinamide and the derivative thereof includes nicotinamide, β-nicotinamide riboside and a salt thereof, β-nicotinamide mononucleotide and a salt thereof, oxidized β-nicotinamide adenine dinucleotide and a salt thereof, reduced β-nicotinamide adenine dinucleotide and a salt thereof, oxidized β-nicotinamide adenine dinucleotide phosphate and a salt thereof or reduced β-nicotinamide adenine dinucleotide phosphate and a salt thereof. The vitamin include vitamin A, vitamin B, vitamin C, vitamin D and vitamin K. The coenzyme include at least one of glutathione and a derivative thereof, adenosine triphosphate and a derivative thereof, pyrroloquinoline quinone and a derivative thereof, adenosylmethionine and a derivative thereof, coenzyme A or a derivative thereof, coenzyme Q or a derivative thereof. Preferably, the nicotinic acid or the derivative thereof in the combination may be β-nicotinic acid riboside and a salt thereof, and β-nicotinic acid mononucleotide and a salt thereof. Preferably, the nicotinamide or the derivative thereof in the combination may be β-nicotinamide riboside and a salt thereof, β-nicotinamide mononucleotide and a salt thereof, oxidized β-nicotinamide adenine dinucleotide and a salt thereof and reduced β-nicotinamide adenine dinucleotide and a salt thereof. Preferably, the vitamin in the combination may be vitamin B, vitamin C and vitamin K. Preferably, the coenzyme in the combination may be glutathione and the derivative thereof and pyrroloquinoline quinone and the derivative thereof. The most important purpose of the combination is to replenish and enhance the level of β-nicotinamide adenine dinucleotide in the gums, so that the inventors propose that the combination comprises β-nicotinamide mononucleotide as the main ingredient, with the other substances included in the combination as auxiliary ingredients.

The inventors propose that the substances in the combination may be added directly to the existing oral care products, and the amount to be added may be 0.1-10% (w/v). According to an embodiment herein, for the oral care composition, β-nicotinamide mononucleotide, a glutathione derivative, S-acetylglutathione, and vitamin C are selected and added to toothpaste. The β-nicotinamide mononucleotide : S-acetylglutathione : vitamin C are mixed in a container in a ratio by weight of 6:2:2 in powder form, based on the total weight of the composition, then added at a dose of 1% of the total weight of the toothpaste and mixed with a spoon until no solids are evident. In another embodiment, the same combination at the same ratio is added to a mouthwash and dissolved. The toothpaste and mouthwash to which the additive combination was added were subjected to comparative tests with the original products in terms of color, odor and stability and antimicrobial properties to demonstrate that the additive combination would not affect the physical and functional properties of the oral care products.

The direct or indirect functional substances in the combination may preferably be β-nicotinamide mononucleotide, β-nicotinamide riboside, oxidized β-nicotinamide adenine dinucleotide, reduced β-nicotinamide adenine dinucleotide, β-nicotinic acid mononucleotide and β-nicotinic acid riboside, more preferably β-nicotinamide mononucleotide, β-nicotinamide riboside and oxidized β-nicotinamide adenine dinucleotide, and even more preferably β -nicotinamide mononucleotide and oxidized β-nicotinamide adenine dinucleotide.

The present invention also provides the use of any of the above oral care additives in the preparation of oral care compositions for the treatment, prevention or relief of oral diseases.

The contents of the present invention are further explained or illustrated below by way of examples, but these examples should not be construed as limiting the scope of protection of the present invention.

### Examples

Where specific conditions are not indicated in the following examples, they are performed under conventional conditions or those recommended by the manufacturers. Percentages as stated are weight percentages unless otherwise noted.

The materials and equipment used in the following examples are described below.
β-nicotinamide mononucleotide: from GeneHarbor (Hong Kong) Biotechnologies Ltd.
Oxidized β-nicotinamide adenine dinucleotide: from GeneHarbor (Hong Kong) Biotechnologies Ltd.
S-acetylglutathione: from GeneHarbor (Hong Kong) Biotechnologies Ltd.
Sodium ascorbate (i.e. Vitamin C): purchased from Shanghai Aladdin Biochemical Technology Co., Ltd.
Darlie Toothpaste: purchased from Market Place by Jasons
Colgate Triple Action Toothpaste: purchased from Market Place by Jasons
Listerine Total Care Mouthwash: purchased from Market Place by Jasons
Powdered gum base: purchased from Glee gum
Sorbitol: purchased from Shanghai Aladdin Biochemical Technology Co., Ltd.
Peppermint extract: purchased from Guangzhou Baiyu Biotechnology Co., Ltd.
Brilliant Blue: purchased from Shandong Gukang Biological Engineering Co., Ltd.
Sodium phosphate (sodium dihydrogen phosphate monohydrate and disodium hydrogen phosphate): purchased from Shanghai Aladdin Biochemical Technology Co., Ltd.

### Example 1: Preparation of a solid oral care additive containing β-nicotinamide mononucleotide

100 g of the solid oral care additive was prepared. In a sterilized beaker of 500 ml, β -nicotinamide mononucleotide, S-acetylglutathione and sodium ascorbate were added in that order, and the weight ratio of the three substances in the oral care additive composition was as follows: β-nicotinamide mononucleotide : S-acetylglutathione : sodium ascorbate = 6:2:2. That is, in 100 g of the solid oral care additive, the weight of β-nicotinamide mononucleotide is 60 g, the weight of S-acetylglutathione is 20 g and the weight of sodium ascorbate is 20 g. The three substances were all in powder form, and upon addition to the beaker, the substances were stirred no less than twenty times with a medicine spoon until they were completely mixed. 10 mg of the mixed sample was dissolved in 1 ml of pure water and the contents of the functional components were analyzed by high performance liquid chromatography in Annex 1.

### Example 2: Preparation of a solid oral care additive containing β-nicotinamide mononucleotide and oxidized β-nicotinamide adenine dinucleotide

100 g of the solid oral care additive was prepared. In a sterilized beaker of 500 ml, β-nicotinamide mononucleotide, oxidized β-nicotinamide adenine dinucleotide, S-acetylglutathione and sodium ascorbate were added in that order, and the weight ratio of the four substances in the oral care additive composition was as follows: β-nicotinamide mononucleotide : oxidized β-nicotinamide adenine dinucleotide : S-acetylglutathione : sodium ascorbate = 3:3:2:2. That is, in 100 g of the solid oral care additive combination, the weight of β-nicotinamide mononucleotide is 30 g, the weight of oxidized β-nicotinamide adenine dinucleotide is 30 g, the weight of S-acetylglutathione is 20 g and the weight of sodium ascorbate is 20 g. The substances were all in powder form, and upon addition to the beaker, the substances were stirred no less than twenty times with a medicine spoon until they were completely mixed. 10 mg of the mixture was dissolved in 1 ml of pure water and the contents of the functional components were analyzed by high performance liquid chromatography in Annex 1.

### Example 3: Preparation of a solid oral care additive containing β-nicotinamide adenine dinucleotide and oxidized β-nicotinamide adenine dinucleotide

100 g of the solid oral care additive was prepared. In a sterilized beaker of 500 ml, nicotinamide adenine dinucleotide, oxidized β-nicotinamide adenine dinucleotide, glutathione and sodium ascorbate were added in that order, and the weight ratio of the four substances in the oral care additive composition was as follows: β-nicotinamide adenine dinucleotide : oxidized β-nicotinamide adenine dinucleotide : S-acetylglutathione : sodium ascorbate = 4:4:1:1. That is, in 100 g of the solid oral care additive, the weight of β-nicotinamide adenine dinucleotide is 40 g, the weight of oxidized β-nicotinamide adenine dinucleotide is 40 g, the weight of glutathione is 10 g and the weight of sodium ascorbate is 10 g. All the four substances were in the form of powder, and upon addition to the beaker, the substances were stirred no less than twenty times with a medicine spoon until they were completely mixed. 10 mg of the mixed sample was dissolved in 1 ml of purified water, and the contents of the functional components were analyzed by high performance liquid chromatography in Annex I.

### Example 4: Preparation of a liquid oral care additive containing β-nicotinamide mononucleotide

100 ml of the liquid oral care additive was prepared. In a sterilized beaker of 500 ml, β-nicotinamide mononucleotide, glutathione and sodium ascorbate were added in that order and the weight ratio of the three substances in the oral care additive composition was as follows: β-nicotinamide mononucleotide : glutathione : sodium ascorbate = 6:2:2, i.e., 600 mg of β-nicotinamide mononucleotide, 200 mg of S-acetylglutathione and 200 mg of sodium ascorbate. All the three substances were in powder form, and upon addition to the beaker, the substances were dissolved in a solution of acidity regulator of edible grade sodium phosphate. 1 g of the oral care additive combination was added to 99 ml of 0.1 M sodium phosphate acidity regulator solution (pH 6.5) and dissolved to form 1% (w/v) of the liquid oral care additive composition. 1 mL of the liquid oral care additive composition was taken to analyze the contents of the functional ingredients by high performance liquid chromatography (HPLC) as described in Annex 1.

### Example 5: Preparation of a liquid oral care additive containing β-nicotinamide adenine dinucleotide

100 ml of the liquid oral care additive was prepared. In a sterilized beaker of 500 ml, β-nicotinamide adenine dinucleotide, glutathione and sodium ascorbate were added in that order, and the weight ratio of the three substances in the oral care additive combination was as follows: β-nicotinamide mononucleotide : glutathione : sodium ascorbate = 4:4:2, i.e., 400 mg of β-nicotinamide mononucleotide, 400 mg of glutathione and 200 mg of sodium ascorbate. All the three substances were in powder form, which were added to the beaker and dissolved in a solution of the acidity regulator (which was an edible grade sodium phosphate). 1 g of the oral care additive combination was added to 99 ml of 0.1 M sodium phosphate acidity regulator solution (pH 6.5) and dissolved to form 1% (w/v) of the liquid oral care additive composition. 1 mL of the liquid oral care additive composition was taken to analyze the content of the functional ingredients by high performance liquid chromatography (HPLC) method in Annex 1.

### Example 6: Preparation of toothpaste containing an oral care additive

100 g of the toothpaste containing the oral care additive was prepared. The solid oral care additive was prepared as in Example 1 and 1 g of the additive was taken and added to a sterilized plastic bag, and then 99 g of Darlie toothpaste was added. Then, the oral care additive combination and the toothpaste were repeatedly mixed in the plastic bag until completely mixed. 1 g of the Darlie toothpaste containing the oral care additive combination was taken and mixed completely in pure water and centrifuged at 13,000 rpm for 5 minutes. The supernatant was taken to analyze the contents thereof by high performance liquid chromatography. The physical odor and taste of the toothpaste containing the oral care additive and the toothpaste without the oral care additive were tested in a double-blind manner. The researcher firstly labeled the two toothpastes as sample 1 and sample 2, then supplied to 10 subjects and compared the color, odor and taste of the two samples. The subjects were asked as to whether there was any difference between sample 1 and sample 2 in terms of the above three sensory perceptions. The results showed that 9 of the 10 subjects found no difference in color, odor and taste between the two samples.

The antimicrobial efficacy of the toothpaste containing the oral care additive and the toothpaste without the oral care additive was also studied. Both toothpastes were tested with *E. coli* according to the method in Annex 2. In a control experiment with three groups, the average diameter of the antibacterial circle of the toothpaste containing the oral care additive was 20.2+1.1, and the average diameter of the antibacterial circle of the toothpaste without the oral care additive was 20.3+1.3. The experimental results showed that the diameters of the antibacterial circle of the toothpaste containing the oral care additive and the toothpaste without the oral care additive were very similar at all the three concentrations as tested, proving that the antibacterial efficacy of the above two toothpastes was the same and that the combination of the oral care additive did not affect the original effectiveness of the toothpaste.

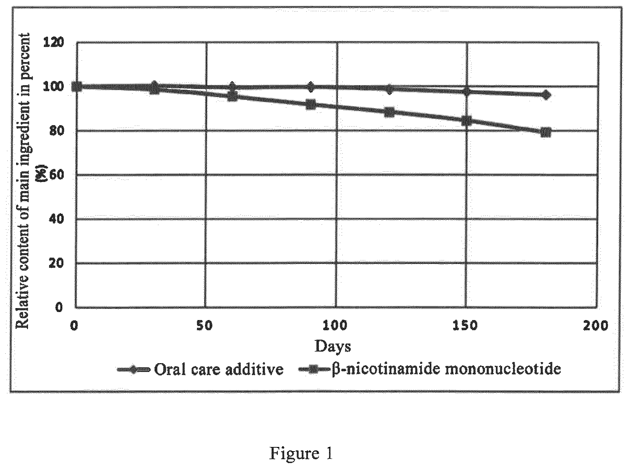

The stability of the toothpaste containing the oral care additive was tested in normal packaging during a six-month experimental period at 25 degrees Celsius and 60% humidity. The content of β-nicotinamide mononucleotide, the main component of the oral care additive combination, was analyzed by high performance liquid chromatography (HPLC) in Annex 1 to study the changes of its content in the toothpaste. The experimental results are shown in FIG. 1 and demonstrate that β-nicotinamide mononucleotide, the main component of the oral care additive, has superior stability in the toothpaste.

### Example 7: Preparation of toothpaste containing an oral care additive

100 g of the toothpaste containing β-nicotinamide mononucleotide as the oral care additive was prepared. 1 g of the additive was taken and added to a sterilized plastic bag, and then 99 g of Darlie toothpaste was added. Then, the oral care additive combination and the toothpaste were repeatedly mixed in the plastic bag until completely mixed. 1 g of the Darlie toothpaste containing the oral care additive combination was taken and mixed completely in pure water and centrifuged at 13,000 rpm for 5 minutes. The supernatant was taken to analyze the content thereof by high performance liquid chromatography. The physical odor and taste of the toothpaste containing the oral care additive and the toothpaste without the oral care additive were tested in a double-blind manner. The researcher firstly labeled the two toothpastes as sample 1 and sample 2, then supplied to 10 subjects and compared the color, odor and taste of the two samples. The subjects were asked as to whether there was any difference between sample 1 and sample 2 in terms of the above three sensory perceptions. The results showed that 9 of the 10 subjects found no difference in color, odor and taste between the two samples.

The stability of the toothpaste containing the oral care additive was tested in normal packaging during a six-month experimental period at 25 degrees Celsius and 60% humidity. The content of β-nicotinamide mononucleotide, the main component of the oral care additive combination, was analyzed by high performance liquid chromatography (HPLC) in Annex 1 to study the changes of its content in the toothpaste. The experimental results are shown in FIG. 1.

### Example 8: Preparation of a mouthwash containing an oral care additive

100 ml of the mouthwash containing the oral care additive was prepared. 1 g of the solid oral care additive was prepared as in Example 1 and added to a sterilized beaker, then 99 g (i.e., about 99 ml in volume) of Listerine Total Care Mouthwash was added, and then the mixture was magnetically stirred at 250 rpm for 30 min in the beaker until completely dissolved. 1 ml of the Listerine Total Care Mouthwash containing the oral care additive was taken to analyze the contents by high performance liquid chromatography in Annex 1.

The physical color, odor and taste of the mouthwash containing the oral care additive and the mouthwash without the oral care additive were tested in double-blind manner. The researcher firstly labeled the two mouthwashes as sample 3 and sample 4, and then supplied to 10 subjects and compared the color, odor and taste of the two samples. The subjects were asked as to whether there was any difference between sample 3 and sample 4 in the above three sensory perceptions. The results showed that 8 of the 10 subjects found no difference in color, odor, and taste between the two samples.

The antibacterial efficacy of the mouthwash containing the oral care additive and the mouthwash without the oral care additive was also studied. Both mouthwashes were tested with *E. coli* according to the method in Annex 3. In a control experiment with three groups, the average diameter of the antibacterial circle of the mouthwash containing the oral care additive was 24.4 mm, and the average diameter of the antibacterial circle of the mouthwash without the oral care additive was 24.1 mm. The experimental results showed that the diameters of the antibacterial circle of the mouthwash containing the oral care additive and the mouthwash without the oral care additive were very close at all the three concentrations as tested, proving that the antibacterial efficacy of the above two was the same and the combination of the oral care additive did not affect the original effectiveness of the mouthwash.

The stability of the mouthwash containing the oral care additive was tested in the original packaging during a six-month experimental period at 25 degrees Celsius and 60% humidity. The content of β-nicotinamide mononucleotide, the main component of the oral care additive, was analyzed by high performance liquid chromatography (HPLC) in Annex 1 to study the changes of its content in the mouthwash. The experimental results showed that the content of β-nicotinamide mononucleotide in the mouthwash after six months was 94.5% of that in the initial sample, which proved the superior stability of β-nicotinamide mononucleotide (the main component of the oral care additive) in the mouthwash.

### Example 9: Preparation of a mint flavored chewing gum containing an oral care additive

200 g of the mint flavored chewing gum containing the oral care additive was prepared. The solid oral care additive was prepared according to the method in Example 1, and 2 g of the additive was taken and mixed completely with 16.5 g of sorbitol and 16.5 g of xylitol by a medicine spoon. 100 g of powdered gum base, 70 g of sorbitol and 15 g of soy lecithin were added to a beaker and heated for 5 minutes at medium heat in a microwave oven. After completion, the mixture was stirred continuously to cool down to 40 degrees Celsius or lower and removed. The pre-mixed powders of the oral care additive, sorbitol and xylitol were piled on a flat surface, and the melted powder gum base was placed in the center of the powder pile. The powder was evenly adhered to the gum by kneading. Afterwards, 0.85 g of the peppermint extract serum and 3-5 drops of Brilliant Blue were added at the same time and the kneading was continued until the gum appeared light blue on an average. The gum was dispersed equally in a 6 cm (L) x 6 cm (W) x 1 cm (H) tray and cut into squares of 20 mm (L) x 20 mm (W) x 1 cm (H) with a knife, and air-dried at room temperature for 12 hours to complete the preparation process. Each of the mint flavored gum containing the oral care additive weighed about 4 g and contained 40 mg of the oral care additive.

### Example 10: Preparation of an oral gel containing an oral care additive

100 g of the oral gel containing the oral care additive was prepared. The solid oral care additive was prepared by the method in Example 1 and 10 g of the additive was taken for use. 15 g of tragacanth gum, 15 g of glycerol and 15 g of sorbitol were added to a beaker in that order, heated to 70 degrees Celsius and stirred continuously until runny and then removed. The mixture was stirred continuously at room temperature and cooled to 40 degrees Celsius, then 10 g of the oral care additive combination, 2.5 g of sodium benzoate and 100 mg of the peppermint extract were added in that order and stirred continuously at room temperature for 12 hr to complete the preparation process.

### Example 11 - Preparation of toothpaste containing an oral care additive

100 g of the toothpaste containing the oral care additive was prepared. 1 g of the oral care additive prepared in Example 2 was added to 99 g of Darlie toothpaste. Then, the oral care additive and the toothpaste were repeatedly mixed in a plastic bag until completely mixed. 1 g of the Darlie toothpaste containing the oral care additive was taken and mixed completely in pure water and centrifuged at 13,000 rpm for 5 minutes. The supernatant was taken to analyze the content thereof by high performance liquid chromatography. The physical odor and taste of the toothpaste containing the oral care additive and the toothpaste without the oral care additive were tested in a double-blind manner. The researcher firstly labeled the two toothpastes as sample 1 and sample 2, then supplied to 10 subjects and compared the color, odor and taste of the two samples. The subjects were asked as to whether there was any difference between sample 1 and sample 2 in terms of the above three sensory perceptions. The results showed that 9 of the 10 subjects found no difference in color, odor and taste between the two samples.

The antimicrobial efficacy of the toothpaste containing the oral care additive and the toothpaste without the oral care additive was also studied. Both toothpastes were tested with *E. coli* according to the method in Annex 2. In a control experiment with three groups, the average diameter of the antibacterial circle of the toothpaste containing the oral care additive was 20.2+1.1, and the average diameter of the antibacterial circle of the toothpaste without the oral care additive was 20.3+1.3. The experimental results showed that the diameters of the antibacterial circle of the toothpaste containing the oral care additive and the toothpaste without the oral care additive were very similar at all three concentrations as tested, proving that the antibacterial efficacy of the above two toothpastes was the same and that the combination of the oral care additive did not affect the original effectiveness of the toothpaste.

The stability of the toothpaste containing the oral care additive was tested in normal packaging during a six-month experimental period at 25 degrees Celsius and 60% humidity. The total content of β-nicotinamide mononucleotide and oxidized β-nicotinamide adenine dinucleotide, the main components of the oral care additive combination, was analyzed by high performance liquid chromatography (HPLC) in Annex 1 to study the changes of the content in the toothpaste. The experimental results showed that the content of β-nicotinamide mononucleotide and oxidized β-nicotinamide adenine dinucleotide in the toothpaste after six months was 96.3% of that in the initial sample, which proved the superior stability of β-nicotinamide mononucleotide and oxidized β-nicotinamide adenine dinucleotide (the main components of the oral care additive combination) in the toothpaste.

### Testing Example

In order to further test the efficacy of the oral care additive combination in oral tissues, a control cell culture test was conducted. HGF-1 cells (Human gingival fibroblast) were selected as the experimental cell model, and the change in cell growth during a time period of 48 hours was compared by means of a control experiment in a medium containing different concentrations (12-50 ug/mL) of the oral care additive versus a blank sample.

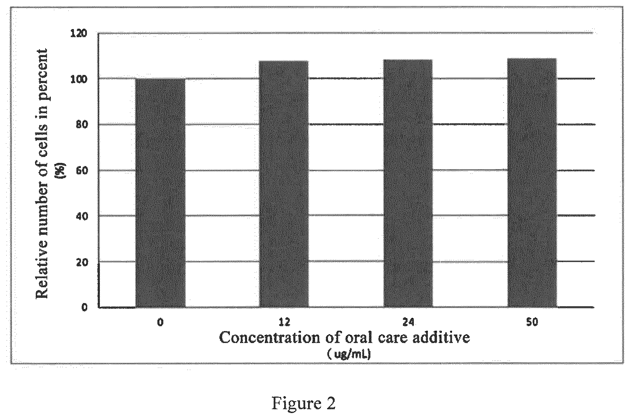

100 g of the oral care additive was prepared according to the method in Example 2. 12 mg, 24 mg and 50 mg of the oral care additive were dissolved respectively into 1000 mL of HGF-1 medium (ATCC-formulated Dulbecco's Modified Eagle's Medium + 10% fetal bovine serum) to form three groups of HGF-1 medium with different concentrations of the oral care additive, and the HGF-1 medium without the oral care additive was used as the blank sample. A 12-well cell culture plate was used and 2.5 × 10⁴ of HGF-1 was added to each well. The plate was placed in a cell culture incubator (37°C, 5% COz) for three hours, and the cell culture medium was withdrawn from the well. The HGF-1 medium with 12 ug/ml oral care additive was added to three wells in a straight row, HGF-1 medium with 24 ug/ml oral care additive was added to three wells in another straight row and HGF-1 medium with 50 ug/ml oral care additive was added to three wells in yet another straight row, and finally, the HGF-1 medium without the oral care additive was added to the three wells in the last straight row. The plate was placed in the cell culture incubator, and the mediums in the sample wells were changed after 24 hours. The average number of cells in the respective mediums was calculated using a cell counter after 48 hours of incubation. In the control test, the average cell count in the HGF-1 medium containing 12 ug/ml oral care additive was 7.8% higher than in the blank sample, and the average cell counts in the HGF-1 mediums containing 24 ug/ml oral care additive and 50 ug/ml oral care additive were 8.4% and 8.8% higher than that in the blank sample, respectively. The results are shown in FIG. 2 and indicate that the oral care additive can promote the growth of the oral tissues and thus can alleviate, prevent or treat the oral diseases, such as gingival inflammation, to a certain extent.

The present invention is not limited by the specific written description above, and the invention may be modified or changed in various ways within the scope set forth in the claims. These changes are within the protection scope of the present invention.

### Annex 1: Conditions for analyzing the contents of the riboside and mononucleotide of nicotinic acid or a derivative thereof (e.g., β-nicotinamide mononucleotide, β-nicotinamide adenine dinucleotide, β-nicotinamide riboside, β-nicotinic acid mononucleotide, nicotinic acid adenine dinucleotide, β-nicotinic acid riboside, etc.) by high performance liquid chromatography

The operation method is as follows:
Solid oral care additive:
   Analyze by high performance liquid chromatography after appropriate dilution with pure water
Liquid oral care additive:
   Analyze by high performance liquid chromatography after appropriate dilution with pure water
Toothpaste:
   Place 10 mg of toothpaste into a small tube, add 1 mL of pure water and mix for 1 min;
centrifuge at 10,000 rpm for 5 min and extract the aqueous phase;
   take 0.5 mL of the aqueous phase and filter it with a 0.22 um syringe filter; and
   analyze by high performance liquid chromatography after appropriate dilution with pure water.
Mouthwash
   Analyze by high performance liquid chromatography after appropriate dilution with pure water.

Analytical conditions for high performance liquid chromatography

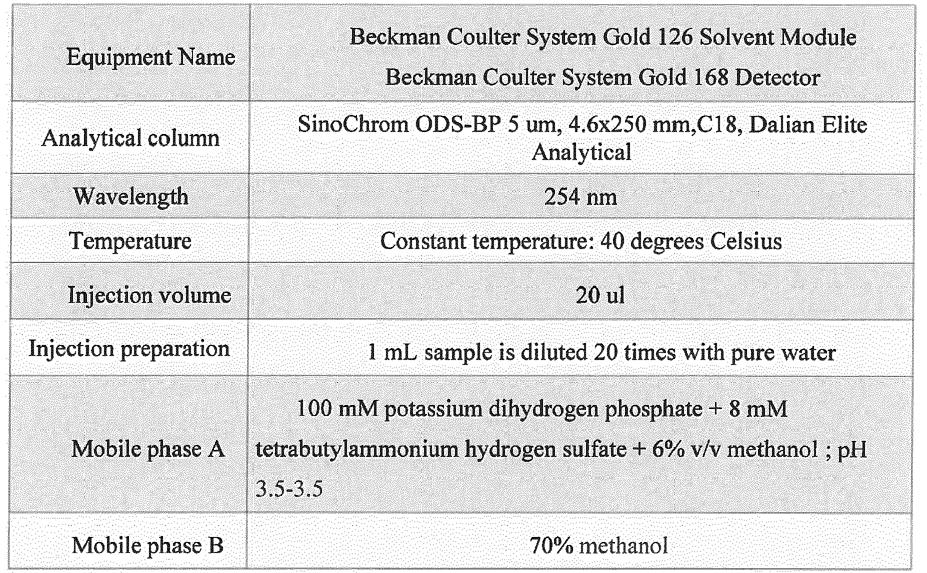

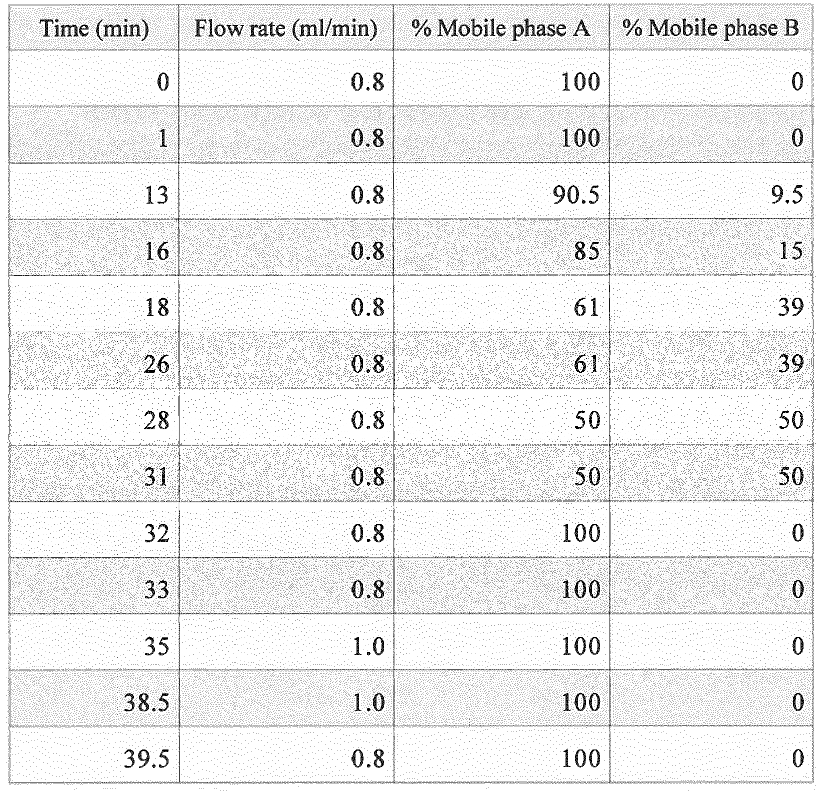

### Annex 2: Antibacterial test for the toothpaste containing an oral care additive combination

*Escherichia coli* is inoculated into LB medium, and after culturing for 24 hours under the conditions of 37 degrees Celsius and 220 rpm, 0.8 mL of the *Escherichia coli* medium is inoculated onto LB agar plate and air-dried for 1 hour. At the center of the plate, a hollow circular hole with a diameter of 5 mm and a depth to the bottom of the plate has been formed by using a sterilized stainless-steel cylindrical rod. 1g of the toothpaste containing the oral care additive combination is taken and mixed thoroughly with 1 mL of sterilized pure water. 0.15 mL of the liquid mixture is added dropwise to the circular hole, and the plate is placed in a constant temperature incubator at 37 degrees Celsius for 24 hours and then removed. The diameter of the antibacterial circle on the plate is measured with a ruler.

### Annex 3: Antibacterial test for the mouthwash containing an oral care additive combination

The *Escherichia coli* is inoculated into LB medium, and after culturing for 24 hours under the conditions of 37 degrees Celsius and 220 rpm, 0.8 mL of *Escherichia coli* medium is inoculated onto LB agar plate and air-dried for 1 hour. At the center of the plate, a hollow circular hole with a diameter of 5 mm and a depth to the bottom of the plate has been formed by using a sterilized stainless-steel cylindrical rod. 1g of the mouthwash containing the oral care additive combination is taken and mixed thoroughly with 1 mL of sterilized pure water. 0.15 mL of the liquid mixture is added dropwise to the circular hole, and the plate is placed in a constant temperature incubator at 37 degrees Celsius for 24 hours and then removed. The diameter of the antibacterial circle on the plate is measured with a ruler.

## Claims

1. An oral care additive **characterized by** comprising at least one main ingredient selected from nicotinic acid or a derivative thereof and nicotinamide or a derivative thereof.

2. The oral care additive according to claim 1, **characterized in that** the nicotinic acid or derivative thereof and the nicotinamide or derivative thereof are selected from β-nicotinic acid riboside and a salt thereof, β-nicotinic acid mononucleotide and a salt thereof, oxidized β-nicotinic acid adenine dinucleotide and a salt thereof, reduced β-nicotinic acid adenine dinucleotide and a salt thereof, oxidized β-nicotinic acid adenine dinucleotide and a salt thereof, reduced β-nicotinic acid adenine dinucleotide phosphate and a salt thereof, oxidized β-nicotinic acid adenine dinucleotide phosphate and a salt thereof, β-nicotinamide riboside and a salt thereof, β-nicotinamide mononucleotide and a salt thereof, oxidized β-nicotinamide adenine dinucleotide and a salt thereof, reduced β-nicotinamide adenine dinucleotide and a salt thereof, oxidized β-nicotinamide adenine dinucleotide phosphate and a salt thereof or reduced β-nicotinamide adenine dinucleotide phosphate and a salt thereof.

3. The oral care additive according to claim 1 or 2, **characterized in that** the oral care additive further comprises an auxiliary ingredient selected from at least one of vitamin and coenzyme.

4. The oral care additive according to claim 3, **characterized in that** the vitamin is selected from at least one of vitamin A, vitamin B, vitamin C, vitamin D and vitamin K.

5. The oral care additive according to claim 3 or 4, **characterized in that** the coenzyme is selected from at least one of glutathione or derivatives thereof, adenosine triphosphate and derivatives thereof, pyrroloquinoline quinone or derivatives thereof, adenosylmethionine or derivatives thereof, coenzyme A or derivatives thereof, coenzyme Q class or derivatives thereof.

6. The oral care additive according to claim 2, **characterized in that** the derivative is β-nicotinic acid riboside or a salt thereof, β-nicotinic acid mononucleotide or a salt thereof, β-nicotinamide riboside or a salt thereof, β-nicotinamide mononucleotide or a salt thereof, oxidized β-nicotinamide adenine dinucleotide or a salt thereof, or reduced β-nicotinamide adenine dinucleotide or a salt thereof;
wherein the vitamin is vitamin B, vitamin C or vitamin K; and
wherein the coenzyme is glutathione or a derivative thereof, or pyrroloquinoline quinone or a derivative thereof.

7. The oral care additive according to any one of claims 1-6, wherein the oral care additive is in a solid, liquid or gaseous form.

8. The oral care additive according to claim 7, **characterized in that** the solid is selected from at least one of powder, paste, gel and crystal, optionally, the liquid is selected from at least one of solution, slurry, essential oil and non-Newtonian fluid; and optionally, the gaseous form is selected from liquefied spray.

9. The oral care additive according to any one of claims 1 to 7, **characterized in that** the main ingredient is present in an amount of 0.1-100% (w/w), preferably 0.1-90% (w/w), and more preferably 0.1-50% (w/w) based on a total weight of the oral care additive.

10. The oral care additive according to claim 3, **characterized in that** the weight ratio of the main ingredient: the vitamin : the coenzyme is (0.1-99%) : (0.1-99%) : (0.1-99%), preferably (0.1-75%) : (0.1-12.5%) : (0.1-12.5%), and more preferably (0.1-50%) : (0.1-25%) : (0.1-25%).

11. An oral care composition **characterized by** comprising the oral care additive according to any one of claims 1-10.

12. The oral care composition according to claim 11, **characterized in that** the oral care additive is present in an amount of 0.1-50% (w/w), preferably 0.1-25% (w/w), and more preferably 0.3-15% (w/w) based on a total weight of said oral care composition.

13. The oral care composition according to claim 11 or 12, **characterized in that** the oral care composition is at least one selected from toothpaste, mouthwash, oral spray and tooth cleaning powder.

14. The oral care composition according to claim 11 or 12, **characterized in that** the oral care composition is a chewing gum or oral gelling agent.

15. The oral care composition according to claim 11 or 12, **characterized in that** the oral care composition is in solid, liquid or gaseous form.

16. The oral care composition according to claim 14, **characterized in that** the chewing gum further comprises at least one of a resin, a wax, a phospholipid, a flavoring agent and an elastomer; and
preferably, the oral gelling agent further comprises glycerin, sorbitol, tragacanth gum, sodium benzoate and peppermint oil.

17. A method of preparing an oral care composition, **characterized by** comprising the steps of
providing an oral care additive comprising at least one main ingredient selected from nicotinic acid or a derivative thereof and nicotinamide or a derivative thereof; and
adding the oral care additive to a carrier that facilitates absorption of the oral care additive into an oral gingival tissue.

18. The method according to claim 17, **characterized in that** said carrier is at least one of a toothpaste, a mouthwash, an oral spray, a tooth cleaning powder, a chewing gum and an oral gelling agent.

19. The method according to claim 17 or 18, **characterized in that** the oral care additive further comprises an auxiliary ingredient selected from at least one of vitamin and coenzyme.

20. The method according to claim 17 or 18, **characterized in that** the nicotinic acid or derivative thereof and the nicotinamide or derivative thereof are selected from β-nicotinic acid riboside and a salt thereof, β-nicotinic acid mononucleotide and a salt thereof, oxidized β-nicotinic acid adenine dinucleotide and a salt thereof, reduced β-nicotinic acid adenine dinucleotide and a salt thereof, oxidized β-nicotinic acid adenine dinucleotide and a salt thereof, reduced β-nicotinic acid adenine dinucleotide phosphate and a salt thereof, oxidized β-nicotinic acid adenine dinucleotide phosphate and a salt thereof, β-nicotinamide riboside and a salt thereof, β-nicotinamide mononucleotide and a salt thereof, oxidized β-nicotinamide adenine dinucleotide and a salt thereof, reduced β-nicotinamide adenine dinucleotide and a salt thereof, oxidized β-nicotinamide adenine dinucleotide phosphate and a salt thereof or reduced β-nicotinamide adenine dinucleotide phosphate and a salt thereof.

21. An oral care kit **characterized by** comprising the oral care composition according to any one of claims 11-16 and a dental gel brace.

22. Use of the oral care additive according to claims 1-10 in the preparation of an oral care composition for treatment, prevention or relief of an oral disease.
